(19) Europäisches Patentamt
European Patent Office
Office européen des brevets

(11) Veröffentlichungsnummer : **0 014 880**
**B1**

(12) **EUROPÄISCHE PATENTSCHRIFT**

(45) Veröffentlichungstag der Patentschrift : **22.09.82**

(21) Anmeldenummer : **80100533.1**

(22) Anmeldetag : **04.02.80**
**Die Akte enthält technische Angaben, die nach dem Eingang der Anmeldung eingereicht wurden und die nicht in dieser Patentschrift enthalten sind.**

(51) Int. Cl.³ : **C 07 C103/48**, C 07 C103/50, C 07 D295/18, C 07 D231/12, C 07 D211/16, A 01 N 39/02, A 01 N 39/04, A 01 N 43/56

(54) **Phenoxycarbonsäureamide, Verfahren zu ihrer Herstellung und ihre Verwendung als Herbizide.**

(30) Priorität : **17.02.79 DE 2906237**

(43) Veröffentlichungstag der Anmeldung : **03.09.80 (Patentblatt 80/18)**

(45) Bekanntmachung des Hinweises auf die Patenterteilung : **22.09.82 Patentblatt 82/38**

(84) Benannte Vertragsstaaten : **BE CH DE FR GB IT NL**

(56) Entgegenhaltungen :
**EP A 0 002 757**
**EP A 0 003 295**
**EP A 0 003 586**
**DE A 2 311 638**
**DE A 2 628 384**
**DE A 2 632 581**
**DE A 2 809 541**

(73) Patentinhaber : **BAYER AG**
**Zentralbereich Patente, Marken und Lizenzen**
**D-5090 Leverkusen 1, Bayerwerk (DE)**

(72) Erfinder : **Förster, Heinz, Dr.**
**Am Eckbusch 47**
**D-5600 Wuppertal-1 (DE)**
Erfinder : **Eue, Ludwig, Dr.**
**Paul-Klee-Strasse 36**
**D-5090 Leverkusen (DE)**
Erfinder : **Schmidt, Robert Rudolf, Dr.**
**Hahnenweg 5**
**D-5000 Köln 80 (DE)**

**EP 0 014 880 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Phenoxycarbonsäureamide, Verfahren zu ihrer Herstellung und ihre Verwendung als Herbizide

Die Erfindung betrifft neue Phenoxycarbonsäureamide, ein Verfahren zu ihrer Herstellung und ihre Verwendung als Herbizide.

Es ist bereits bekannt geworden, daß bestimmte Phenoxycarbonsäure-Derivate herbizide Eigenschaften besitzen. So lassen sich z.B. der 5-(2-Chlor-4-trifluormethylphenoxy)-2-nitro-α-phenoxy-propionsäure-methylester und der 5-(2-Chlor-4-trifluormethyl-phenoxy)-2-nitro-α-phenoxy-propionsäure-ethylester zur Unkrautbekämpfung verwenden (vgl. US-PS 4 093 446 und DE-OS 2 311 638). Die Wirkung dieser Stoffe ist sowohl bei einem Einsatz nach dem Pre-emergence- als auch bei einem Einsatz nach dem Post-emergence-Verfahren gut. Nachteilig ist jedoch, daß einige Problemunkräuter und Ungräser nicht immer voll bekämpft werden.

Weiterhin ist bereits bekannt, daß Diphenylether, die in einem Phenylring in 4-Position durch Trifluormethyl substituiert sind und an diesem Phenylring gegebenenfalls noch zusätzliche Halogenatome in 2- und 6-Stellung tragen und im anderen Phenylring einen Oxyalkancarbonsäureamid-Rest in der 3-Position aufweisen, jedoch in der zur Trifluormethylgruppe entgegengesetzten para-Position nicht durch Nitro substituiert sind, zur Unkrautbekämpfung geeignet sind (vgl. DE-OS 28 09 541). Die Wirksamkeit dieser Stoffe läßt aber in manchen Fällen zu wünschen übrig.

Schließlich wurde schon beschrieben, daß solche Diphenylether, die zwar in dem einen Phenylring eine Nitrogruppe in der 4-Position und in der benachbarten 3-Position einen Oxyalkancarbonsäurerest aufweisen, in dem anderen Phenylring aber nicht durch Trifluormethyl substituiert sind, herbizide Eigenschaften besitzen (vgl. DE-OS 26 32 581). Allerdings ist auch die Wirksamkeit dieser Stoffe nicht immer ausreichend.

Es wurden nun neue Phenoxycarbonsäureamide der Formel

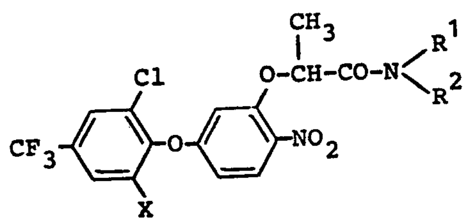

(I)

in welcher

$R^1$ für Wasserstoff oder Alkyl mit 1 bis 3 Kohlenstoffatomen steht,

$R^2$ für Alkoxycarbonylmethyl mit 1 bis 4 Kohlenstoffatomen in der Alkoxygruppe, Aminocarbonylmethyl, Di-$C_{1-4}$-alkylamino-carbonylmethyl oder für gegebenenfalls ein- oder zweifach durch Alkyl mit 1 bis 3 Kohlenstoffatomen, Alkoxy mit 1 bis 3 Kohlenstoffatomen, Methylthio, Chlor und/oder Nitro substituiertes Phenyl steht, oder

$R^1$ und $R^2$ zusammen mit dem angrenzenden Stickstoffatom für jeweils gegebenenfalls durch 1 oder 2 Methyl- oder Ethylgruppen substituiertes Pyrrolidinyl oder Morpholinyl, für jeweils gegebenenfalls durch 1 bis 3 Methyl- oder Ethylgruppen substituiertes Piperidyl, Indolyl, Tetrahydroindolyl, Perhydroindolyl, Tetrahydrochinolyl, Tetrahydroisochinolyl, Perhydrochinolyl, Perhydroisochinolyl, Perhydrothiazolyl oder Perhydroazepinyl oder für jeweils gegebenenfalls durch $C_1$-$C_3$-Alkyl, $C_1$-$C_3$-Alkoxy, $C_1$-$C_3$-Alkylthio, Phenyl, Chlor, Brom, Jod, Trifluormethyl, Cyano, Acetyl, Carbomethoxy oder Carbethoxy substituiertes Pyrrolyl, Pyrazolyl, Imidazolyl, 1,2,4-Triazol-1-yl, 1,2,3-triazol-1-yl, 1,3,4-triazol-1-yl oder 1,2,3,4-tetrazol-1-yl stehen und

X für Wasserstoff oder Chlor steht, gefunden.

Weiterhin wurde gefunden, daß man die Phenoxycarbonsäureamide der Formel (I) erhält, wenn man Phenoxycarbonsäurechloride der Formel

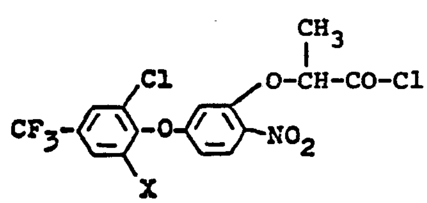

(II)

in welcher X die oben angegebene Bedeutung hat, mit Verbindungen der Formel

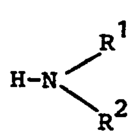

(III)

in welcher $R^1$ und $R^2$ bzw. der Rest

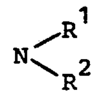

die oben angegebene Bedeutung haben, gegebenenfalls in Gegenwart eines Säureakzeptors und gegebenenfalls in Gegenwart eines Verdünnungsmittels umsetzt.

Schließlich wurde gefunden, daß die neuen Phenoxycarbonsäureamide der Formel (I) starke herbizide Eigenschaften aufweisen.

Überraschenderweise zeigen die erfindungsgemäßen Phenoxycarbonsäureamide eine wesentlich bessere herbizide Wirkung als die aus dem Stand der Technik bekannten Verbindungen 5-(2-Chlor-4-trifluormethyl-phenoxy)-2-nitro-α-phenoxypropionsäure-methylester und 5-(2-Chlor-4-trifluormethylphenoxy)-2-nitro-α-phenoxy-propionsäure-ethylester (vgl. US-PS 4 093 446 und DE-OS 2 311 638), welche die chemisch nächstliegenden Wirkstoffe gleicher Wirkungsrichtung sind. Besonders hervorzuheben ist, daß die erfindungsgemäßen Phenoxycarbonsäureamide vorteilhafter zur Bekämpfung von Problemunkräutern und -Ungräsern, wie z.B. Galium und Cyperus, geeignet sind als die oben erwähnten vorbekannten Stoffe.

Weiterhin übertreffen die erfindungsgemäßen Stoffe in ihrer herbiziden Wirksamkeit auch das konstitutionell ähnliche 5-(2,4-Dichlor-phenoxy)-2-nitro-α-phenoxypropionsäure-ethylamid, das in der DE-OS 26 32 581 offenbart ist. Eine derartige Überlegenheit konnte im Hinblick auf den bekannten Stand der Technik nicht vorausgesagt werden.

In der DE-OS 28 09 541 werden Diphenylether beschrieben, die sich konstitutionell von den erfindungsgemäßen Substanzen in eindeutiger Weise unterscheiden. So enthalten die in der DE-OS 28 09 541 offenbarten Verbindungen in der Position, die der Trifluormethylgruppe entgegengesetzt ist, in keinem Fall die für die erfindungsgemäßen Stoffe charakteristische Nitrogruppe. Das Substitutionsmuster dieser vorbekannten Stoffe weicht also ganz erheblich von demjenigen der erfindungsgemäßen Diphenylether ab.

Verwendet man 5-(2,6-Dichlor-4-trifluormethyl-phenoxy)-2-nitro-α-phenoxy-propionsäure-chlorid und 1,2,4-Triazol als Ausgangsstoffe, so kann der Reaktionsablauf nach dem erfindungsgemäßen Verfahren durch das folgende Formelschema wiedergegeben werden

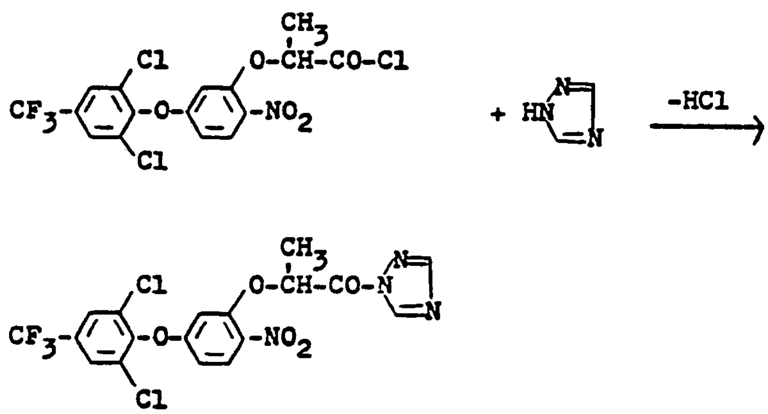


Die bei der Durchführung des erfindungsgemäßen Verfahrens als Ausgangsstoffe benötigten Phenoxycarbonsäurechloride sind durch die Formel (II) definiert. In dieser Formel steht X für diejenigen Rest, die bereits im Zusammenhang mit der Beschreibung der erfindungsgemäßen Stoffe der Formel (I) für diesen Rest genannt wurden.

Als Beispiele für die Phenoxycarbonsäurechloride der Formel (II) seien genannt:

5-(2-Chlor-4-trifluormethyl-phenoxy)-2-nitro-α-phenoxy- und 5-(2,6-Dichlor-4-trifluormethyl-phenoxy)-2-nitro-α-phenoxy-propionsäurechlorid.

Die Phenoxycarbonsäurechloride der Formel (II) sind bekannt oder lassen sich nach üblichen Methoden in einfacher Weise herstellen (vgl. US-PS 4 093 446). So lassen sich Phenoxycarbonsäurechloride der Formel (II) synthetisieren, indem man Phenoxycarbonsäuren der Formel

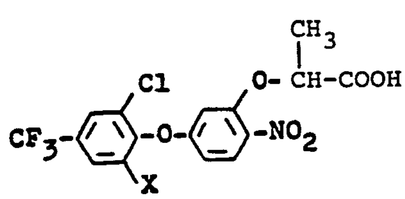

(IV)

in welcher X die oben angegebene Bedeutung hat, mit Chlorierungsmitteln wie z.B. Thionylchlorid, gegebenenfalls unter Verwendung von Verdünnungsmitteln wie z.B. Benzol oder Ethylenchlorid, bei Temperaturen zwischen 10 und 100 °C umsetzt und destillativ leicht flüchtige Komponenten nach dem Ende der Reaktion entfernt.

Die Phenoxycarbonsäuren der Formel (IV) sind ebenfalls bekannt oder lassen sich nach üblichen Methoden in einfacher Weise herstellen (vgl. US-PS 4 093 446). So lassen sich Phenoxycarbonsäuren der Formel (IV) synthetisieren, indem man Phenoxycarbonsäureester der Formel

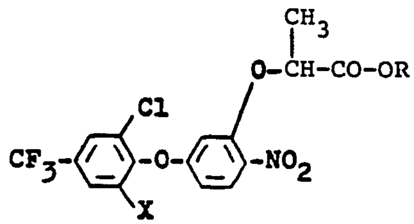

(V)

in welcher

X die oben angegebene Bedeutung hat und

R für Alkyl (insbesondere für Methyl oder Ethyl) steht,

mit wäßrigen Alkalihydroxidlaugen, vorzugsweise mit Natronlauge oder Kalilauge, welche gegebenenfalls mit organischen Lösungsmitteln wie z.B. Methanol, Ethanol oder Dioxan verdünnt sind, bei Temperaturen zwischen 20 und 100 °C umsetzt.

Als Beispiele für die Phenoxycarbonsäuren der Formel (IV) seien genannt :

5-(2-Chlor-4-trifluormethyl-phenoxy)-2-nitro-α-phenoxy und 5-(2,6-Dichlor-4-trifluoromethyl-phenoxy)-2-nitro-α-phenoxy-propionsäure.

Die Phenoxycarbonsäureester der Formel (V) sind ebenfalls bekannt oder lassen sich nach üblichen Methoden in einfacher Weise herstellen (vgl. US-PS 4 093 446). So lassen sich Phenoxycarbonsäureester der Formel (V) synthetisieren, indem man Phenol-Derivate der Formel

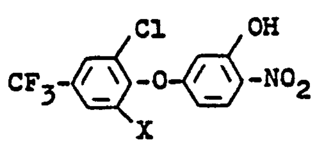

(VI)

in welcher X die oben angegebene Bedeutung hat, bzw. die Natrium- oder Kalium-Salze dieser Phenol-Derivate mit α-Halogen-carbonsäureestern der Formel

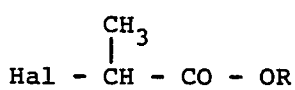

(VII)

in welcher

R für Alkyl (insbesondere für Methyl oder Ethyl) steht und

Hal für Chlor oder Brom steht, gegebenenfalls in Gegenwart eines Säurebindemittels wie z.B. Natriummethylat oder Kaliumcarbonat und gegebenenfalls unter Verwendung eines polaren Verdünnungsmittels wie z.B. Methanol, Acetonitril oder Sulfolan, bei Temperaturen zwischen 20 und 100 °C umsetzt.

Als Beispiele für die Phenoxycarbonsäureester der Formel (V) seien genannt :

5-(2-Chlor-4-trifluormethyl-phenoxy)-2-nitro-α-phenoxy- und 5-(2,6-Dichlor-4-trifluormethyl-phenoxy)-2-nitro-α-phenoxy-propionsäuremethylester und -ethylester.

Die Phenolderivate der Formel (VI) sind bekannt (vergleiche US-PS 4 093 446). Als Beispiele für die Phenolderivate der Formel (VI) seien genannt :

5-(2-Chlor-4-trifluormethyl-phenoxy)-2-nitro-phenol und 5-(2,6-Dichlor-4-trifluormethyl-phenoxy)-2-nitrophenol.

Bekannt sind auch die α-Halogen-carbonsäureester der Formel (VII). Als Beispiele hierfür seien genannt :

α-Chlor-propionsäuremethylester und -ethylester sowie α-Brom-propionsäuremethylester und -ethylester.

Die bei dem erfindungsgemäßen Verfahren weiterhin als Ausgangsstoffe benötigten Verbindungen

sind durch die Formel (III) allgemein definiert. In dieser Formel stehen $R^1$ und $R^2$ für diejenigen Reste, die bereits im Zusammenhang mit der Beschreibung der Formel (I) für diese Reste genannt wurden.

Die Verbindungen der Formel (III) sind bekannt. Als Beispiele seien im einzelnen aufgeführt:

Aminoessigsäure-methylester, -ethylester, -propylester und -butylester, Anilin, 2-Methyl-, 3-Methyl- und 4-Methyl-anilin, 4-Methoxy-, 3,4-Dimethoxy- und 4-Methylthio-anilin, 2-Chlor-, 3-Chlor- und 4-Chloranilin, 2,4-Dichlor-, 3,4-Dichlor-, 2,5-Dichlor- und 3,5-Dichlor-anilin, 2-Nitro-, 3-Nitro- und 4-Nitro-anilin, 3-Nitro-6-methyl-anilin; N-Methyl-anilin, 2-Methyl-, 3-Methyl- und 4-Methyl-N-methyl-anilin, 4-Methoxy-, 3,4-Dimethoxy- und 4-Methyl-thio-N-methyl-anilin, 2-Chlor-, 3-Chlor- und 4-Chlor-N-methyl-anilin, 2,4-Dichlor-, 3,4-Dichlor-, 2,5-Dichlor- und 3,5-Dichlor-N-methyl-anilin, 2-Nitro-, 3-Nitro- und 4-Nitro-N-methyl-anilin, 3-Nitro-6-methyl-N-methyl-anilin; N-Ethyl-anilin, 2-Methyl-, 3-Methyl- und 4-Methyl-N-ethyl-anilin, 4-Methoxy-, 3,4-Dimethoxy- und 4-Methyl-thio-N-ethyl-anilin, 2-Chlor-, 3-Chlor- und 4-Chlor-N-ethyl-anilin; 2,4-Dichlor-, 3,4-Dichlor-, 2,5-Dichlor- und 3,5-Dichlor-N-ethyl-anilin, 2-Nitro-, 3-Nitro- und 4-Nitro-N-ethyl-anilin, 3-Nitro-6-methyl-N-ethyl-anilin;

N-n-Propyl-anilin, 2-Methyl-, 3-Methyl- und 4-Methyl-N-n-propyl-anilin, 4-Methoxy-, 3,4-Dimethoxy- und 4-Methylthio-N-n-propyl-anilin, 2-Chlor-, 3-Chlor-, und 4-Chlor-N-n-propyl-anilin, 2,4-Dichlor-, 3,4-Dichlor-, 2,5-Dichlor- und 3,5-Dichlor-N-n-propyl-anilin, 2-Nitro-, 3-Nitro- und 4-Nitro-N-n-propyl-anilin; 3-Nitro-6-methyl-N-n-propyl-anilin; N-iso-Propyl-anilin, 2-Methyl-, 3-Methyl- und 4-Methyl-N-iso-propyl-anilin, 4-Methoxy-, 3,4-Dimethoxy- und 4-Methyl-thio-N-iso-propyl-anilin, 2-Chlor-, 3-Chlor- und 4-Chlor-N-iso-propyl-anilin, 2,4-Dichlor-, 3,4-Dichlor-, 2,5-Dichlor- und 3,5-Dichlor-N-iso-propyl-anilin, 2-Nitro-, 3-Nitro- und 4-Nitro-N-iso-propyl-anilin, 3-Nitro-6-methyl-N-iso-propyl-anilin; Pyrrolidin, 2-Methyl-pyrrolidin, Morpholin, 2,6-Dimethyl-morpholin, Piperidin, 2-Methyl- und 4-Methyl-piperidin, 2,4-Dimethyl-piperidin, 2,4,6-Trimethyl-piperidin, 2-Ethyl-piperidin, 2-Methyl-5-ethyl-piperidin, Tetra-hydroindolin, 2-Methyltetrahydroindolin, 2,3,3-Trimethyl-tetrahydroindolin, Perhydroindolin, 2-Methylperhydroindolin, 2,2-Dimethyl-perhydroindolin, Tetra-hydrochinolin, Perhydrochinolin, 4-Methyl- und 6-Methyl-perhydrochinolin, Tetrahydroisochinolin, Perhydrothiazol, Perhydroazepin (Hexamethylenimin);

Pyrrol, 2,4-Dimethyl- und 2,5-Dimethyl-pyrrol, Tetramethylpyrrol, 3(5)-Methylpyrazol, 4-Methylpyrazol, 3(5)-Ethylpyrazol, 4-Ethylpyrazol, 3(5)-Isopropylpyrazol, 4-Isopropylpyrazol, 3,5-Dimethylpyrazol, 3,5-Dimethyl-4-acetylpyrazol, 3,4,5-Trimethyl-pyrazol, 3(5)-Phenylpyrazol, 4-Phenylpyrazol, 3,5-Diphenylpyrazol, 3(5)-Phenyl-5(3)-methyl-pyrazol, 3(5)-Chlorpyrazol, 4-Chlorpyrazol, 4-Brom-pyrazol, 4-Jodpyrazol, 3,4,5-Trichlorpyrazol, 3,4,5-Tribrompyrazol, 3,5-Dimethyl-4-chlorpyrazol, 3,5-Dimethyl-4-brompyrazol, 4-Chlor-3(5)-methylpyrazol, 4-Brom-3(5)-methylpyrazol, 4-Methyl-3,5-dichlor-pyrazol, 3(5)-Methyl-4,5(3)-dichlorpyrazol, 3(5)-Chlor-5(3)-methylpyrazol, 4-Methoxypyrazol, 3(5)-Methyl-5(3)-methoxypyrazol, 3(5)-Ethoxy-4,5(3)-dimethylpyrazol, 3(5)-Methyl-5(3)-trifluormethyl-pyrazol, 3,5-Bistrifluormethylpyrazol, 3(5)-Methyl-5(3)-carbethoxypyrazol, 3,5-Biscarbethoxypyrazol, 3,4,5-Triscarbethoxypyrazol, 3(5)-Methyl-5(3)-methylthio-4-carbethoxypyrazol, 4-Methyl-3,5-biscarbethoxypyrazol, 4-Cyanopyrazol, 4-Methoxy-3,5-Dichlorpyrazol, Imidazol, 2,4,5-Trichlorimidazol, 1,2,4-Triazol, 3(5)-Methyl-1,2,4-triazol, 3,5-Dimethyl-1,2,4-triazol, 3(5)Chlor-1,2,4-triazol, 3(5)-Brom-1,2,4-triazol, 3(5)-Chlor-5(3)-methyl-1,2,4-triazol, 3,5-Dichlor-1,2,4-triazol, 3,5-Dibrom-1,2,4-triazol, 3(5)Chlor-5(3)-cyano-1,2,4-triazol, 3(5)-Chlor-5(3)-phenyl-1,2,4-triazol, 3(5)-Chlor-5(3)-carbomethoxy-1,2,4-triazol, 3(5)-Methylthio-1,2,4-triazol, 4(5)-Methyl-1,2,3-triazol, 4,5-Dimethyl-1,2,3-triazol, 4(5)-Phenyl-1,2,3-triazol, 4(5)-Chlor-1,2,3-triazol, 1,2,3-Triazol-4(5)-yl-carbonsäureethylester, 1,2,3-Triazol-4,5-yl-dicarbonsäuredimethylester, 5-Methyltetrazol, 4-Chlor-tetrazol, Tetrazolyl-5-carbonsäureethylester, 1,3,4-Triazol und 1,2,3,4-Tetrazol.

Das erfindungsgemäße Verfahren wird bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Ether wie Diethyl- und Dibutylether, Tetrahydrofuran und Dioxan, Ketone wie Aceton, Methylethyl-, Methylisopropyl- und Methylisobutylketon, Nitrile wie Acetonitril und Propionitril, Ester wie Essigsäuremethylester oder -äthylester sowie Formamide wie Dimethylformamid.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethyl-anilin, Dimethylbenzylamin und Pyridin.

An Stelle der oben genannten Säureakzeptoren können auch die in diesem Falle in entsprechendem Überschuß einzusetzenden Reaktanden der Formel (III) als Säurebindemittel fungieren.

Die Reaktionstemperatur kann innerhalb eines größeren Bereichs variiert werden. Im allgemeinen arbeitet man zwischen −20 und 100 °C, vorzugsweise zwischen 0 und 50 °C. Das erfindungsgemäße Verfahren wird im allgemeinen bei Normaldruck durchgeführt.

Bei der Durchführung des erfindungsgemäßen Verfahrens setzt man auf 1 Mol Phenoxycarbonsäurechlorid der Formel (II) im allgemeinen 1 bis 3 Mol, vorzugsweise 1 bis 1,5 Mol einer Verbindung der Formel (III) sowie gegebenenfalls 1 bis 2 Mol Säurebindemittel ein. Die Umsetzung wird im allgemeinen in einem geeigneten Verdünnungsmittel durchgeführt und das Reaktionsgemisch wird mehrere Stunden bei der erforderlichen Temperatur gerührt. Die Isolierung der erfindungsgemäßen Reaktionsprodukte der Formel (I) erfolgt nach üblichen Methoden. Im allgemeinen wird das Reaktionsgemisch nach beendeter Umsetzung mit einem mit Wasser nicht mischbaren Lösungsmittel wie z.B. Toluol verdünnt, nacheinander mit verdünnter Salzsäure oder Schwefelsäure, verdünnter Natronlauge und Wasser gewaschen. Die nichtwässrige Phase wird dann getrocknet, filtriert und durch Destillation bei vermindertem Druck vom Lösungsmittel befreit.

Die Produkte fallen im allgemeinen in fester Form an und können durch Umkristallisation gereinigt werden. Zu ihrer Charakterisierung dient der Schmelzpunkt.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden :

Dikotyle Unkräuter der Gattungen : Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen : Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen : Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen : Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen übergeführt werden, wie Lösungen, Emulsionen, Suspensionen, Pulver, Schäume, Pasten, Granulate, Wirkstoffimprägnierte Natur- und synthetische Stoffe und Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkokole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser.

Als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie

Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die gute herbizide Wirksamkeit der erfindungsgemäßen Phenoxycarbonsäureamide geht aus den nachfolgenden Beispielen hervor. In den Beispielen werden die nachstehend angegebenen Verbindungen als Vergleichssubstanzen eingesetzt

(A) = 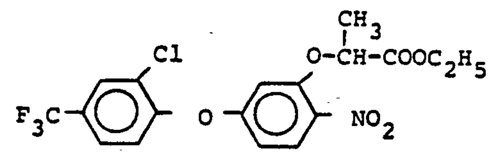

(B) = 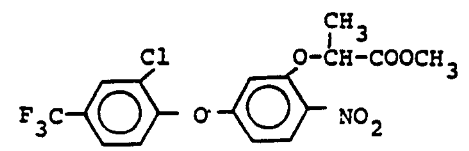

Beispiel A

Pre-emergence-Test

Lösungsmittel : 5 Gewichtsteile Aceton
Emulgator : 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung .

In diesem Test zeigt der im Beispiel (3) aufgeführte erfindungsgemäße Wirkstoff eine bessere Wirkung gegen Cyperus als die bekannte Vergleichssubstanz (A).

Beispiel B

Pre-emergence-Test

Lösungsmittel : 5 Gewichtsteile Aceton
Emulgator : 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten :

0 % = keine Wirkung (wie unbehandelte Kontrolle)
100 % = totale Vernichtung

In diesem Test zeigt der im Beispiel (3) aufgeführte erfindungsgemäße Wirkstoff eine bessere Wirkung gegen Galium als die bekannte Vergleichssubstanz (B).

## Herstellungsbeispiele

### Beispiel 1

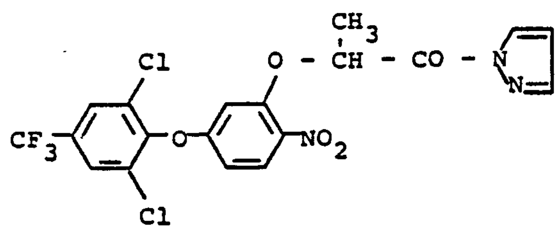

Eine Lösung von 22,9 g (50 mMol) 5-(2,6-Dichloro-4-trifluormethyl-phenoxy)-2-nitro-α-phenoxy-propionsäurechlorid in 40 ml Toluol wird zu einer auf 0 bis 5 °C gekühlten Lösung von 3,6 g (53 mMol) Pyrazol und 5,6 g (55 mMol) Triethylamin in 70 ml Toluol tropfenweise gegeben. Die Reaktionsmischung wird über Nacht bei Raumtemperatur gerührt, mit Toluol verdünnt, neutral gewaschen, getrocknet, filtriert und eingeengt. Man erhält 20 g (81,6 % der Theorie) 5-(2,6-Dichlor-4-trifluormethyl-phenoxy)-2-nitro-α-phenoxy-propionsäure-pyrazolid in Form gelber Kristalle vom Schmelzpunkt 144 °C.

Analog Beispiel 1 werden die in der nachstehenden Tabelle 1 aufgeführten Verbindungen der Formel (I) hergestellt :

Tabelle 1

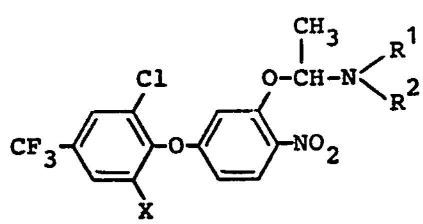 (I)

| Beispiel Nr. | X | 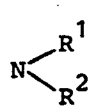 | Schmelzpunkt (°C) |
|---|---|---|---|
| 2 | H | 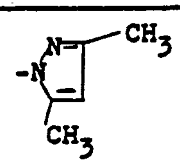 | 97 |
| 3 | H | 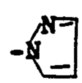 | 115 |
| 4 | H | 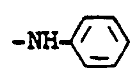 | 106 |
| 5 | H | 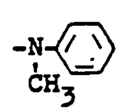 | 112 |

| Beispiel Nr. | X | 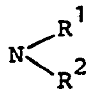 | Schmelzpunkt (°C) |
|---|---|---|---|
| 6 | H | 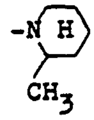 | 104 |
| 7 | H | $-NH-CH_2-CO-OC_2H_5$ | 82 |
| 8 | Cl | $-NH-CH_2-CO-OC_2H_5$ | 119 |

Die als Ausgangsstoffe zu verwendenden Phenoxycarbonsäurechloride können beispielsweise wie folgt hergestellt werden :

Beispiel II-1

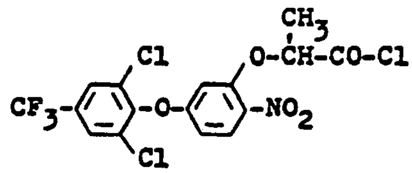

9,7 g (82 mMol) Thionylchlorid werden bei Raumtemperatur tropfenweise zu einer Lösung von 30 g (68 mMol) 5-(2,6-Dichlor-4-Trifluormethyl-phenoxy)-2-nitro-α-phenoxy-propionsäure und 0,5 ml Dimethylformamid in 100 ml 1,2-Dichlorethan gegeben. Die Mischung wird 4 Stunden unter Rückfluß erhitzt. Man gibt Aktivkohle dazu, filtriert und engt ein. Nach dem Digerieren des öligen Rückstandes mit 100 ml Ligroin erhält man 26,2 g (84 % der Theorie) 5-(2,6-Dichlor-4-trifluor-methyl-phenoxy)-2-nitro-α-phenoxy-propion-säurechlorid in Form gelber Kristalle vom Schmelzpunkt 92 °C.

Die als Vorprodukte benötigten Phenoxycarbonsäuren können beispielsweise wie folgt hergestellt werden :

Beispiel IV-1

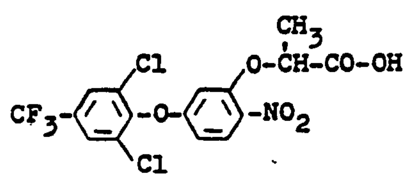

135 g (0,3 Mol) 5-(2,6-Dichloro-4-trifluormethyl-phenoxy)-2-nitro-α-phenoxy-propionsäuremethylester und 30 ml konzentrierte wässrige Natronlauge werden in 400 ml Acetonitril und 150 ml Wasser 24 Stunden bei 20 °C gerührt. Die Lösung wird eingeengt, der Rückstand in 500 ml Wasser aufgenommen und mit 50 ml konzentrierter Salzsäure angesäuert. Man erhält 93 g (71 % der Theorie) 5-(2,6-Dichlor-4-trifluormethyl-phenoxy)-2-nitro-α-phenoxy-propionsäure in Form blaßgelber Kristalle vom Schmelzpunkt 146 °C (nach Umkristallisation aus Toluol/Cyclohexan).

Die als Vorprodukte benötigten Phenoxycarbonsäureester können wie folgt hergestellt werden :

Beispiel V-1

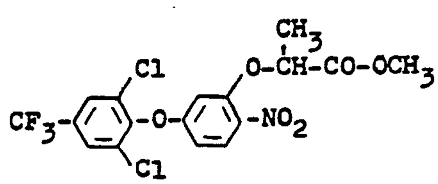

40 g (0,24 Mol) α-Bromo-propionsäuremethylester werden tropfenweise zu einer auf 50 °C erwärmten Mischung aus 73,6 g (0,2 Mol) 5-(2,6-Dichlor-4-trifluormethyl-phenoxy)-2-nitro-phenol, 32 g Kaliumcarbonat und 200 ml Acetonitril gegeben. Das Reaktionsgemisch wird 5 Stunden unter Rückfluß erhitzt, dann in 1 l Wasser gegossen und mit 1 l Toluol extrahiert. Die Toluolphase wird mit 300 ml in wässriger Natronlauge und dann mit 500 ml Wasser gewaschen. Nach Abdestillieren des Lösungsmittels im Vakuum erhält man als Öl, welches bei Zugabe von Methanol kristallisiert, 74 g (81 % der Theorie) 5-(2,6-Dichlor-4-trifluormethyl-phenoxy)-2-nitro-α-phenoxy-propionsäuremethylester mit dem Schmelzpunkt 78 °C.

**Ansprüche**

1. Phenoxycarbonsäureamide der Formel

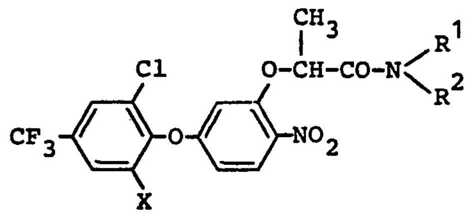

(I)

in welcher

$R^1$ für Wasserstoff oder Alkyl mit 1 bis 3 Kohlenstoffatomen steht,

$R^2$ für Alkoxycarbonylmethyl mit 1 bis 4 Kohlenstoffatomen in der Alkoxygruppe, Aminocarbonylmethyl, Di-$C_{1-4}$-alkylamino-carbonylmethyl oder für gegebenenfalls ein- oder zweifach durch Alkyl mit 1 bis 3 Kohlenstoffatomen, Alkoxy mit 1 bis 3 Kohlenstoffatomen, Methylthio, Chlor und/oder Nitro substituiertes Phenyl steht, oder

$R^1$ und $R^2$ zusammen mit dem angrenzenden Stickstoffatom für jeweils gegebenenfalls durch 1 oder 2 Methyl- oder Ethylgruppen substituiertes Pyrrolidinyl oder Morpholinyl, für jeweils gegebenenfalls durch 1 bis 3 Methyl- oder Ethylgruppen substituiertes Piperidyl, Indolyl, Tetrahydroindolyl, Perhydroindolyl, Tetrahydrochinolyl, Tetrahydroisochinolyl, Perhydrochinolyl, Perhydroisochinolyl, Perhydrothiazolyl oder Perhydroazepinyl oder für jeweils gegebenenfalls durch $C_1$-$C_3$-Alkyl, $C_1$-$C_3$-Alkoxy, $C_1$-$C_3$-Alkylthio, Phenyl, Chlor, Brom, Jod, Trifluormethyl, Cyano, Acetyl, Carbomethoxy oder Carbethoxy substituiertes Pyrrolyl, Pyrazolyl, Imidazolyl, 1,2,4-Triazol-1-yl, 1,2,3-triazol-1-yl, 1,3,4-triazol-1-yl oder 1,2,3,4-tetrazol-1-yl stehen und

X für Wasserstoff oder Chlor steht.

2. Phenoxycarbonsäureamid der Formel

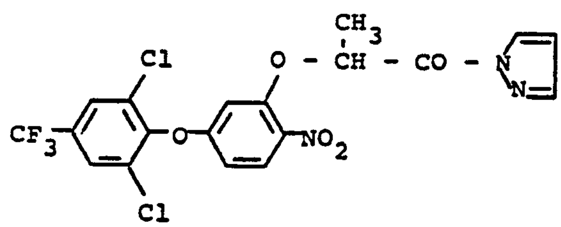

3. Phenoxycarbonsäureamid der Formel

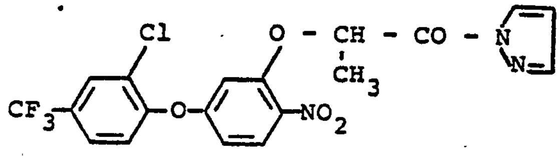

4. Phenoxycarbonsäureamid der Formel

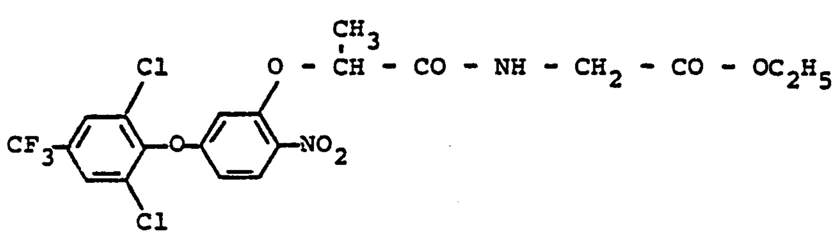

5. Verfahren zur Herstellung von Phenoxycarbonsäureamiden der Formel (I), dadurch gekennzeichnet, daß man Phenoxycarbonsäurechloride der Formel

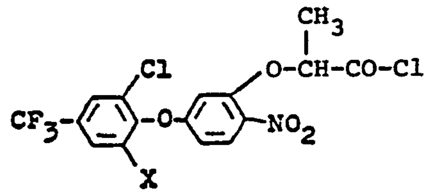

(II)

in welcher X die oben angegebene Bedeutung hat, mit Verbindungen der Formel

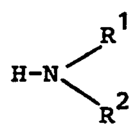

(III)

in welcher $R^1$ und $R^2$ bzw. der Rest

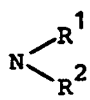

die oben angegebene Bedeutung haben, gegebenenfalls in Gegenwart eines Säureakzeptors und gegebenenfalls in Gegenwart eines Verdünnungsmittels umsetzt.

6. Herbizide Mittel, gekennzeichnet durch einen Gehalt an mindestens einem Phenoxycarbonsäureamid der Formel (I).

7. Verwendung von Phenoxycarbonsäureamiden der Formel (I) zur Bekämpfung von Unkräutern.

8. Verfahren zur Herstellung von herbiziden Mitteln, dadurch gekennzeichnet, daß man Phenoxycarbonsäureamide der Formel (I) mit Streckmitteln und/oder oberflächenaktiven Stoffen vermischt.

**Claims**

1. Phenoxycarboxylic acid amides of the formula

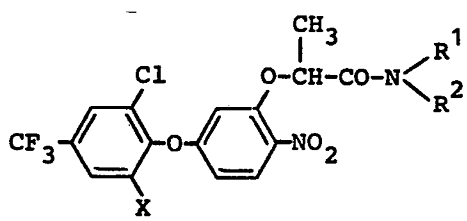

(I)

in which

$R^1$ represents hydrogen or alkyl with 1 to 3 carbon atoms,

$R^2$ represents alkoxycarbonylmethyl with 1 to 4 carbon atoms in the alkoxy group, aminocarbonylmethyl, di-$C_{1-4}$-alkylamino-carbonylmethyl or phenyl which is optionally monosubstituted or disubstituted by alkyl with 1 to 3 carbon atoms, alkoxy with 1 to 3 carbon atoms, methylthio, chlorine and/or nitro, or

$R^1$ and $R^2$, together with the adjacent nitrogen atom, represent pyrrolidinyl or morpholinyl, in each case optionally substituted by 1 or 2 methyl or ethyl groups, piperidyl, indolyl, tetrahydroindolyl, perhydroindolyl, tetrahydroquinolyl, tetrahydroisoquinolyl, perhydroquinolyl, perhydroisoquinolyl, perhydrothiazolyl or perhydroazepinyl, in each case optionally substituted by 1 to 3 methyl or ethyl groups, or pyrrolyl, pyrazolyl, imidazolyl, 1,2,4-triazol-1-yl, 1,2,3-triazol-1-yl, 1,3,4-triazol-1-yl or 1,2,3,4-tetrazol-1-yl, in each case optionally substituted by $C_1$-$C_3$-alkyl, $C_1$-$C_3$-alkoxy, $C_1$-$C_3$-alkylthio, phenyl, chlorine, bromine, iodine, trifluoromethyl, cyano, acetyl, carbomethoxy or carbethoxy and

X represents hydrogen or chlorine.

2. Phenoxycarboxylic acid amide of the formula

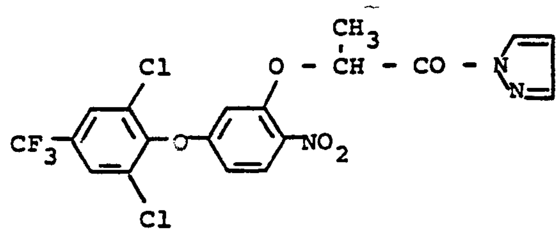

3. Phenoxycarboxylic acid amide of the formula

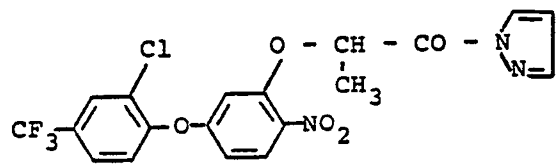

4. Phenoxycarboxylic acid amide of the formula

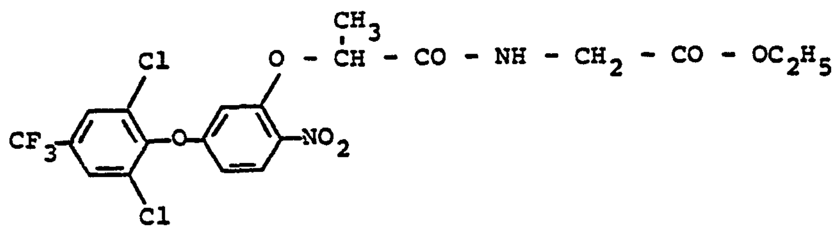

5. Process for the preparation of phenoxycarboxylic acid amides of the formula (I), characterised in that phenoxycarboxylic acid chlorides of the formula

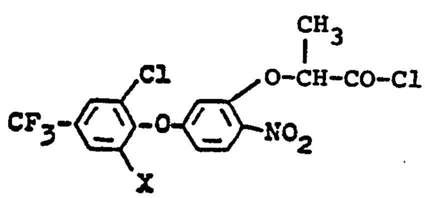 (II)

in which X has the meaning indicated above, are reacted with compounds of the formula

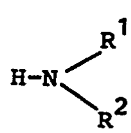 (III)

in which $R^1$ and $R^2$ or the radical

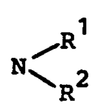

have the meaning indicated above, if appropriate in the presence of an acid acceptor and if appropriate in the presence of a diluent.

6. Herbicidal agents, characterised in that they contain at least one phenoxycarboxylic acid amide of the formula (I).

7. Use of phenoxycarboxylic acid amides of the formula (I) for combating weeds.

8. Process for the preparation of herbicidal agents, characterised in that phenoxycarboxylic acid amides of the formula (I) are mixed with extenders and/or surface-active substances.

**Revendications**

1. Amides d'acides phénoxycarboxyliques de formule

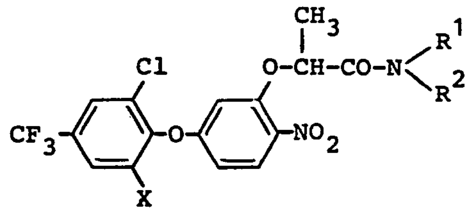

(I)

dans laquelle

$R^1$ représente un atome d'hydrogène ou un groupe alkyle contenant 1 à 3 atomes de carbone,

$R^2$ représente un groupe alcoxycarbonylméthyle contenant 1 à 4 atomes de carbone dans le groupe alcoxy, un groupe aminocarbonylméthyle, un groupe di-alkyl (en $C_1$-$C_4$) aminocarbonylméthyle ou un groupe phényle éventuellement substitué une ou deux fois par un groupe alkyle contenant 1 à 3 atomes de carbone, par un groupe alcoxy contenant 1 à 3 atomes de carbone, par un groupe méthylthio, par un atome de chlore et/ou par un groupe nitro, ou

$R^1$ et $R^2$, ensemble avec l'atome d'azote adjacent, représentent un groupe morpholinyle ou un groupe pyrrolidinyle éventuellement substitué chaque fois par un ou deux groupes méthyle ou éthyle, un groupe perhydro-azépinyle, perhydrothiazolyle, perhydro-isoquinolyle, perhydroquinolyle, tétrahydro-isoquinolyle, perhydroquinolyle, perhydroindolyle, tétrahydro-indolyle, indolyle ou pipéridyle éventuellement substitué chaque fois par un à trois groupes méthyle ou éthyle, ou encore un groupe 1,2,3,4-tétrazol-1-yle, 1,3,4-triazol-1-yle, 1,2,3-triazol-1-yle, 1,2,4-triazol-1-yle, imidazolyle, pyrazolyle ou pyrrolyle éventuellement substitué chaque fois par un groupe alkyle en $C_1$-$C_3$, un groupe alcoxy en $C_1$-$C_3$, un groupe alkyl (en $C_1$-$C_3$)-thio, un groupe phényle, un atome de chlore, un atome de brome, un atome d'iode, un groupe trifluorométhyle, un groupe cyano, un groupe acétyle, un groupe carbométhoxy ou un groupe carbéthoxy, et

X représente un atome d'hydrogène ou de chlore.

2. Amide d'acide phénoxycarboxylique de formule

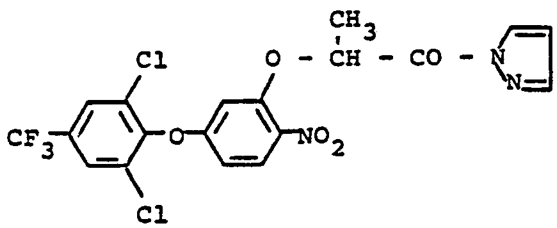

3. Amide d'acide phénoxycarboxylique de formule

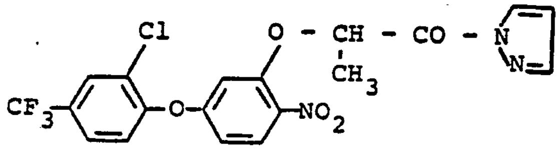

4. Amide d'acide phénoxycarboxylique de formule

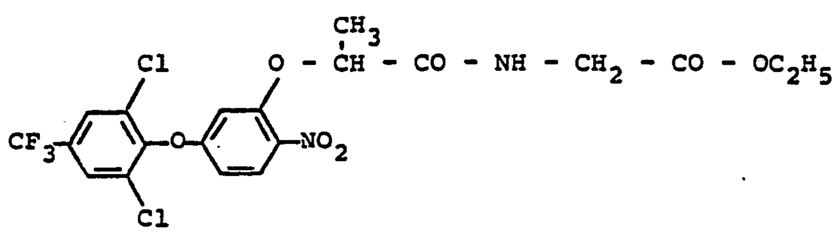

5. Procédé de préparation d'amides d'acides phénoxycarboxyliques de formule (I), caractérisé en ce qu'on fait réagir des chlorures d'acides phénoxycarboxyliques de formule

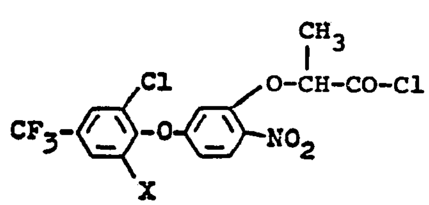

(II)

dans laquelle X a la signification indiquée ci-dessus, avec des composés de fcrmule

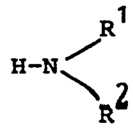

(III)

dans laquelle $R^1$ et $R^2$ ou le radical

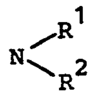

ont les significations indiquées ci-dessus, éventuellement en présence d'un accepteur d'acide et éventuellement en présence d'un diluant.

6. Agents herbicides, caractérisés en ce qu'ils contiennent au moins un amide d'acide phénoxycarboxylique de formule (I).

7. Utilisation d'amides d'acides phénoxycarboxyliques de formule (I) en vue de combattre les plantes adventices.

8. Procédé de préparation d'agents herbicides, caractérisé en ce qu'on mélange des amides d'acides phénoxycarboxyliques de formule (I) avec des diluants et/ou des substances tensio-actives.